(19) Europäisches Patentamt
European Patent Office
Office européen des brevets

(11) EP 2 029 673 B1

(12) **EUROPEAN PATENT SPECIFICATION**

(45) Date of publication and mention of the grant of the patent:
**17.11.2010 Bulletin 2010/46**

(21) Application number: **07725771.5**

(22) Date of filing: **01.06.2007**

(51) Int Cl.:
***C09B 11/08*** *(2006.01)* ***C07K 1/13*** *(2006.01)*
***G01N 33/68*** *(2006.01)* ***G01N 33/50*** *(2006.01)*
***G01N 33/542*** *(2006.01)*

(86) International application number:
**PCT/EP2007/004891**

(87) International publication number:
**WO 2007/144077 (21.12.2007 Gazette 2007/51)**

(54) **IMPROVED PHOTOSTABLE FRET-COMPETENT BIARSENICAL-TETRACYSTEINE PROBES BASED ON FLUORINATED FLUORESCEINS**

VERBESSERTE PHOTOSTABILE FRET-KOMPETENTE BIARSENTETRACYSTEINSONDEN AUF BASIS FLUORIERTER FLUORESZEINE

SONDES DE TETRACYSTEINE BIARSENICALES COMPETENTES POUR UNE USURE PHOTOSTABLE AMELIOREE BASEES SUR DES FLUORESCEINES FLUOREES

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **12.06.2006 EP 06012026**

(43) Date of publication of application:
**04.03.2009 Bulletin 2009/10**

(73) Proprietor: **Max-Planck-Gesellschaft zur Förderung der Wissenschaften e.V.**
**80539 München (DE)**

(72) Inventors:
- **JARES-ERIJMAN, Elisabeth A.**
  **1428 Buenos Aires (AR)**
- **SPAGNUOLO, Carla C.**
  **Martinez (1640) Buenos Aires (AR)**
- **VERMEIJ, Rolf J.**
  **7534 MH Glanerbrug (NL)**
- **JOVIN, Thomas M.**
  **37077 Göttingen (DE)**

(74) Representative: **Schneider, Peter Christian**
**Fiedler, Ostermann & Schneider**
**Patentanwälte**
**Obere Karspüle 41**
**37073 Göttingen (DE)**

(56) References cited:
**WO-A-2005/040197** **US-A- 6 008 378**
**US-A1- 2006 229 441**

- **W.C.SUN ET AL.: "Synthesis of Fluorinated Fluoresceins" J.ORG.CHEM., vol. 62, 1997, pages 6469-6475, XP002468936 cited in the application**
- **S.R.ADAMS ET AL.: "New Biarsenical Ligands and Tetracysteine Motifs for Protein Labeling in Vitro and in Vivo: Synthesis and Biological Applications" J.AM.CHEM.SOC., vol. 124, no. 21, 2002, pages 6063-6076, XP002468937 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 029 673 B1

## Description

The invention relates to fluorogenic dyes, complexes of such fluorogenic dyes and a protein with a tetracysteine motif, a method of synthesizing such fluorogenic dyes, a method of examining a sample using such fluorogenic dyes, and a use of such fluorogenic dyes.

Reference is made to the following documents of prior art:

(1) Griffin, B. A.; Adams, S. R.; Tsien, R. Y., Science 1998, 281, 269-272.

(2) Sosinsky, G.; Gaietta, G.; Giepmans, B.; Deerinck, T.; Adams, S.; Hand, G.; Mackey, M.; Terada, M.; Smock, A.; Tsien, R. Y.; Ellisman, M., Biophys. J. 2005, 88, 31A-32A.

(3) Tour, O.; Meijer, R. M.; Zacharias, D. A.; Adams, S. R.; Tsien, R. Y., Nat. Biotechnol. 2003, 21, 1505-1508.

(4) Hoffmann, C.; Gaietta, G.; Bunemann, M.; Adams, S. R.; Oberdorff-Maass, S.; Behr, B.; Vilardaga, J. P.; Tsien, R. Y.; Eisman, M. H.; Lohse, M. J., Nat. Methods 2005, 2, 171-176.

(5) Jares-Erijman, E. A.; Jovin, T. M., Nat. Biotechnol. 2003, 21, 1387-1395.

(6) Selvin, P.R., Annu. Rev. Biophys. Biomol. Struct. 2002, 31, 275-302.

(7) Martin, B. R.; Giepmans, B. N. G.; Adams, S. R.; Tsien, R. Y., Nat. Biotechnol. 2005, 23, 1308-1314.

(8) Adams, S. R.; Campbell, R. E.; Gross, L. A.; Martin, B. R.; Walkup, G. K.; Yao, Y.; Llopis, J.; Tsien, R. Y., J. Am. Chem. Soc. 2002, 124, 6063-6076; see also US 6008378.

(9) Sun, W. C.; Gee, K. R.; Klaubert, D. H.; Haugland, R. P., J. Org. Chem. 1997, 62, 6469-6475.

(10) Lindqvist, L., Arkiv. Kemi 1961, 16, 79-138.

(11) Sun, W. C.; Gee, K. R.; Klaubert, D. H.; Haugland, R. P., Synthesis of fluorinated fluoresceins. J. Org. Chem. 1997, 62, (19), 6469-6475.

(12) Adams, S. R.; Campbell, R. E.; Gross, L. A.; Martin, B. R.; Walkup, G. K.; Yao, Y.; Llopis, J.; Tsien, R. Y., New biarsenical Ligands and tetracysteine motifs for protein labeling in vitro and in vivo: Synthesis and biological applications. J. Am. Chem. Soc. 2002, 124, (21), 6063-6076.

(13) Jovin, T. M.; Arndt-Jovin, D. J.; Marriott, G.; Clegg, R. M.; Robert-Nicaud, M.; Schormann, T. Optical Microscopy for Biology 1990, 575-602.

Biarsenical ligands are membrane-permeable fluorogenic dyes, which form highly stable complexes with tetracysteine motifs engineered in a target protein of interest.[1] The probes and targets are small compared to visible fluorescent proteins, thereby reducing potential stereochemical interference while providing (i) controllable times of delivery in pulse chase experiments,[2] and (ii) special reactivities enabling chromophore assisted light inactivation experiments (CALI)[3] and non-fluorescent readouts.[2] Furthermore, the combination of biarsenical dyes with visible fluorescence proteins (VFPs) as Förster resonance energy transfer[4] donor-acceptor (DA) pairs constitutes a very attractive technology for the assessment of conformational changes and molecular interactions in living cells. Due to the inverse 6$^{th}$ power distance dependence of FRET, the range of separations that can be determined with confidence about the Förster critical distance for 50% FRET efficiency ($R_o$) is very narrow.[5,6] The generation of DA pairs with large $R_o$ is essential for extending the range over which FRET is operative.

Although optimization of the biarsenical binding motif has led to significant improvements in affinity and signal levels,[7] the limited photostability and pH sensitivity of fluorescein derivatives in the physiological range constitute inherent limitations that still preclude their widespread application.

It is an object of the current invention to provide improved fluorogenic dyes. More specific it is an object of the current invention to provide an improved biarsenical tetracyctein probe exhibiting significant improvements in important properties over the original fluorescein derivative FlAsH, in particular a higher absorbance, larger Stokes shift, higher quantum yield, higher photostability, and reduced pH dependence.

It is a further object of the invention to provide a suitable method of synthesizing such improved dyes.

It is a still further object of the current invention to provide an examining method using such improved dyes.

It is a still further object of current invention to provide an advantageous use for such improved dyes.

In one aspect of the invention there is provided a fluorogenic dye having the formula

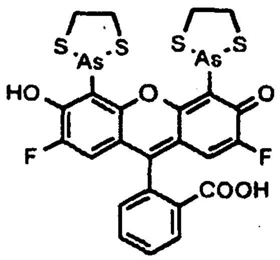

(F2FlAsH).

and salts of said fluorogenic dye.

Another important new dye is

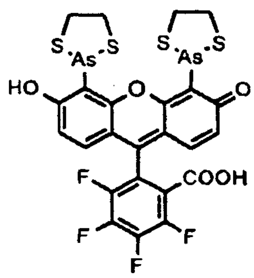

(F4FlAsH)

Fluoro-substituted versions, F2FlAsH and F4FlAsH, are introduced, exhibiting significant improvements in important properties over the original fluorescein derivative FlAsH[8]. Compared to FlAsH, F2FlAsH has higher absorbance, larger Stokes shift, higher quantum yield, higher photostability, and reduced pH dependence. The emission of F4FlAsH lies in a region intermediate to that of FlAsH and ReAsH (a resorufin biarsenical),[8] providing a new color and excellent luminosity. In addition, the two new probes form a new FRET pair with a substantially larger $R_o$ value than any obtained with these dyes (see below).

Syntheses of the tetrafluorinated (F4FlAsH-$EDT_2$) and difluorinated FlAsH (F2FlAsH-$EDT_2$) derivatives most advantageously is accomplished from the parent halogenated fluoresceins according to [4]. In particular the synthesis comprises the steps of

- reacting

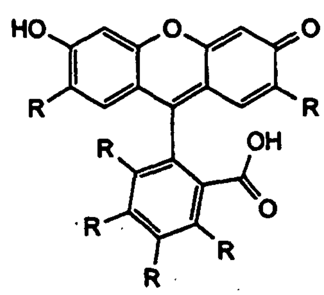

with HgO to form

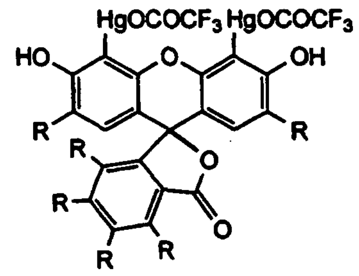

;

- reacting

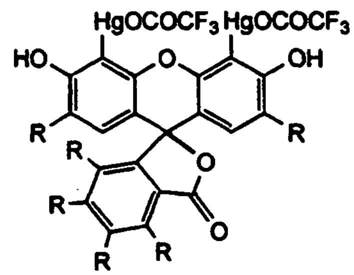

with $AsCl_3$ and EDT to form

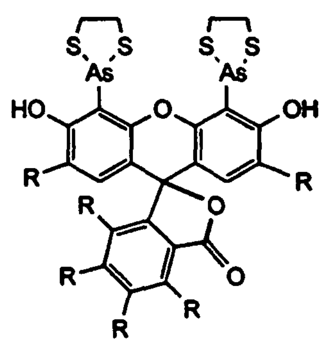

,

wherein R = H, F.

The absorption maximum of F2FlAsH is shifted 11 nm to the blue, compared to FlAsH, whereas the maximum of F4FlAsH is displaced 17 nm to the red (Fig. 1, Table 1). Upon formation of a complex of F2FlAsH with a 12-mer peptidic sequence (FLNCCPGCCMEP, P12) as a model target,[7] fluorescence is observed with an emission peak at 522 nm. Thus, the Stokes shift is 22 nm, 7 nm greater than that of the FlAsH complex. The fluorescence intensity ($\lambda_{exc}$ 490 nm, $\lambda_{em}$ 525 nm) is 4-fold that of the complex with the parent FlAsH probe. This enhancement is attributable to a larger extinction coefficient at 490 nm (2x), and a greater emission quantum yield (2x). The radiative lifetime of F2FlASH-P12 (4.78 ns) is similar to that of the corresponding FlAsH complex (4.88 ns, Table 1).

The emission peak of F4FlAsH-P12 at 544 nm expands the spectral range of the biarsenical dyes. In addition, the fluorescence lifetime increases to a value (5.2 ns). The two fluorinated derivatives provide new combinations with FRET donors and acceptors within and outside of the biarsenical family.

*Table 1*. Photophysical data for the biarsenical-P12 complexes

| | $\lambda_{abs}$ [nm] | $\lambda_{em}$ [nm] | $\varepsilon_{max}$ [$M^{-1}cm^{-1}$] | $\tau$ [ns] | $k_{b1}$ [$s^{-1}$] | $pb^a$ [%] |
|---|---|---|---|---|---|---|
| FlAsH-P12 | 511 | 527 | 52000 | 4.88 | $3.2 \cdot 10^4$ | 87 |
| F2FlAsH-P12 | 500 | 522 | 65500 | 4.78 | $6.2 \cdot 10^2$ | 32 |
| F4FlAsH-P12 | 528 | 544 | 35100 | 5.18 | $9.1 \cdot 10^3$ | 89 |
| [a] photobleaching: loss of fluorescence after 120 min of irradiation. | | | | | | |

It has been reported that fluorination of fluorescein[9] leads to greater resistance to photobleaching to a degree that depends on the number of fluorine atoms. The inventors observed a 50× increase in photostability of the 2',7'-difluoroderivative, whereas for the tetrafluoroderivative, the photostability is similar to that of the parent dye-complex (Fig. 2, Table 1). The fluorine atoms in positions 2' and 7', presumably lead to a reduction in lifetime of the triplet state that serves as an intermediate in the photobleaching process.[10]

To evaluate the sensitivity to pH, the probes were dissolved in phosphate buffers ranging in pH from 7.8 to 5.6. FlAsH-P12 displayed a 50% decrease at the absorption peak of the dianion responsible for fluorescence, whereas the absorption of F2FlAsH-P12, and F4FlAsH-P12 only decreased by 16% (Figure S2; Supporting information). The complexes of the fluorinated dyes exhibited a corresponding brighter emission at lower pH. These results were expected in view of the lower pKs of the dianion and monoanion forms of the parent fluorinated fluoresceins.[9]

The $R_o$ values of F2FlAsH, F4FlAsH, FlAsH and ReAsH in different donor-acceptor combinations are given in Table 2. F2FlAsH and F4FlAsH have a very favorable spectral overlap, leading to a large J (the overlap integral) and thus an $R_o$ of 5.4 nm, greater than any of the values obtained with the other combinations of donors and acceptors based on biarsenical dyes. For example the FlASH-ReAsH pair has an $R_o$ of 3.9 nm. Thus, the new F2FlAsH- F4FlAsH probes constitute a new donor acceptor pair extending the dynamic range for heteroFRET in studies of living cells by 40%. They also provide new possibilities in combination with other FRET donors and acceptors within and outside the biarsenical family.

*Table 2.* Critical Förster distances

| | Acceptor | | | |
|---|---|---|---|---|
| | ReAsH | | F4FlAsH | |
| Donor | $10^{14} \cdot J$ | $R_o$ nm $(\Phi_d)^a$ | $10^{13} \cdot J$ | $R_o$ nm $(\Phi_d)$ |
| FlAsH | 5.03 | 3.9 (0.4) [b] | 1.48 | 4.7 (0.4) |
| F2FlAsH | 3.44 | 4.1 (0.8) | 1.58 | 5.4 (0.8) |
| F4FlAsH | 6.45 | 4.1 (0.4) | 1.17 | 4.5 (0.4) |
| | F2FlAsH | | FlAsH | |
| | $10^{13} \cdot J$ | $R_o$ nm $(\Phi_d)$ | $10^{14} \cdot J$ | $R_o$ nm $(\Phi_d)$ |
| F2FlAsH | 1.37 | 5.2 (0.8) | 6.41 | 4.1 (0.4) |
| [a] Emission quantum yield. [b] Approximate value from [8]. | | | | |

The F2FlAsH-F4FlAsH DA pair was employed in a titration of a F2FlAsH complex of biotin-P12 bound to streptavidin, with the same peptide bearing F4FlAsH (Figure 3). Upon saturation of the free sites remaining on the tetrameric streptavidin, the FRET efficiency was 0.34, corresponding to an apparent mean computed transfer distance of 5.0 nm.

An important consideration related to quantitative FRET determinations based on VFPs is the restricted motion of the chromophore inside the β-barrel of the protein. In order to accurately estimate distances by FRET, one requires knowledge of the relative orientation of the dyes. The general assumption of the value 2/3 for the orientation factor $\kappa^2$ only applies if both donor and acceptor are in rapid, isotropic rotational motion. This is impossible for VFPs due to their mass (27 Kda); the rotational correlation times are much longer than the fluorescence lifetimes.

The fluorescence anisotropies determined for F2FlAsH-P12 biotin, and F4FlAsH-P12 biotin (1 μM in 20 mM HEPES, pH 7.4) were 0.038, and 0.046, respectively, implying that one can apply the 2/3 $\kappa^2$ value with confidence in the case of small and/or mobile targets, thereby allowing accurate distance determinations by FRET. Rotational motion may be restricted in larger proteins, thereby enabling FRET measurements by homotransfer.[5]

In conclusion, we present two new derivatives of the FlAsH family, one of them with 50× improved photostability, lower pH sensitivity, higher absorbance and quantum yield, and the second adding a new color to the palette of biarsenical dyes. In addition, the two compounds form an excellent FRET pair with a large critical distance, facilitating improved structural and dynamic studies of living cells.

To provide a better understanding of the current invention materials and methods are explained below in detail. Further, for better illustration some drawings are provided in which

Figure 1 illustrates dye structures, and absorption and emission (□) spectra of F2FlAsH-P12 (-) and F4FlAsH-P12 (- -);

Figure 2 illustrates a graph illustrations photobleaching of F2FlAsH-P12 (○), F4FlAsH- P12 (□) and FlAsH-P12 (+). Full lines correspond to exponential fits. Complexes of the three dyes (10 μM) were irradiated with a mercury arc lamp through a 490-560 nm filter with an irradiance of 70 $mW/cm^2$;

Figure 3 illustrates titration of a complex of streptavidin (0.8 μM)-biotinylated F2FlAsH-P12 (0.8 μM), with biotinylated F4FlAsH-P12 (spectrally unmixed data). Inset: relative change in donor emission (at 520 nm);

Figure 4 illustrates fluorescence emission spectra of biarsenical-peptide complexes (0.1μM). F2FlAsH-P12 ($\lambda_{exc}$ 490 nm); F4FlAsH-P12 ($\lambda_{exc}$ 520 nm); FlAsH-P12 ($\lambda_{exc}$ 490 nm);

Figure 5 illustrates the dependence of the absorption (left panel) and emission (right panel) properties within the physiological pH range (5.7 - 7.8);

Figure 6 illustrates photobleaching recorded during 120 min. of irradiation and monitored by fluorescence emission. $\lambda_{exc}$ 490 nm. **a.** FlAsH- P12; **b.** F2FlAsH-P12; **c.** F4FlAsH-P12;

## 1 - Materials

**General Procedures.** NMR measurements were carried out on a Bruker 200 MHz AM, 400 MHz, 500 MHz AMX, Mercury 300 MHz (Varian) or on a INOVA 500 MHz (Varian) NMR spectrometer. Chemical shifts are in ppm (internal reference TMS) and coupling constants are given in Hz.

4-Fluororesorcinol was purchased from CPC Scientific, San Jose, CA, USA and model peptide from WITA GmbH, Germany. All other chemicals were obtained from Aldrich Chem. Co.

Reactions were monitored by thin-layer chromatography on Merck silica gel plates (60F-254). Column chromatography was performed on silica gel (230-400 mesh, Merck ASTM) or on Fluorisil® (60-100 mesh, J.T.Baker).

ESI and HRMS were measured on an APEX IV 7 Tesla-Fourier Transform Ion Cyclotron Resonance (FTICR)-Mass spectrometer (Bruker) or on a TSQ 7000 Triple-Stage-Quadrupol-Instrument (Finnigan) with Electrospray-Ionisation, at the Georg-August-University of Goettingen, Germany.

Absorption and fluorescence spectra were measured with a UVIKON 943 Double Beam UV/Vis absorption spectrophotometer and with a Perkin Elmer LS50B fluorescence spectrophotometer, respectively. Lifetime measurements were made by TCSPC in a Horiba Jobin Yvon IBH Fluorescence Lifetime Spectrometer System. The excitation source at 495 nm was a NanoLED N-01 Aqua and the detection module was TBX-04-A. Irradiation was carried out using a Superlite SUV-DC-P system incorporating a 200W DC Super-Pressure short arc lamp coupled to a light guide for high UV transmission and an electronic timer for exposure time control (Lumatec GmbH, Munich, Germany). Output light was passed through a band-pass filter Schott BG18.

## 2 - Synthetic Methods

### General Synthesis of Biarsenical Derivatives.

3,4,5,6-tetrafluorofluorescein, 2',7'-difluorofluorescein and FlAsH derivatives were prepared according to [11, 12].

**Fluorescein-4',5'-bis(mercuric diacetate).** Fluorescein

(1 g, 3 mmol) was added to a stirred solution of HgO (2.6 g, 12 mmol) in trifluoroacetic acid (40 mL). The reaction was mantained at room temperature overnight, added to water and the precipitate was collected by filtration and dried *in vacuo* over $P_2O_5$. Yield, 2.5 g (87%). The crude product was used without further purification.

**2',7'-difluorofluorescein-4',5'-bis(mercuric diacetate).**

2',7'-difluorofluorescein (0.3 g, 0.8 mmol) was added to a stirred solution of HgO (0.37 g, 1.7 mmol) in trifluoroacetic acid (6.5 mL). The reaction was mantained at room temperature overnight, added to water and the precipitate was collected by filtration and dried *in vacuo* over $P_2O_5$. Yield, 0.65 g (80%). The crude product was used without further purification.

**3,4,5,6-tetrafluorofluorescein-4',5'-bis(mercuric diacetate).**

3,4,5,6-tetrafluorofluorescein (0.5 g, 1.13 mmol) was added to a stirred solution of HgO (0.52 g, 2.4 mmol) in trifluoroacetic acid (9 mL). The reaction was mantained at room temperature overnight, the precipitate was collected by filtration and dried *in vacuo* over $P_2O_5$. Yield, 1.10 g (95%). The crude product was used without further purification.

**4',5'-Bis(1,2,3-dithioarsolan-2-yl)-fluorescein, FlAsH-EDT$_2$.**

Crude Fluorescein-4',5'-bis(mercuric trifluoroacetate) (92.3 mg, 0.1 mmol) was suspended in dry NMP (1.5 mL) under Ar and treated with arsenic trichloride (0.16 mL, 2.0 mmol), DIPEA (0.14 mL, 0.80 mmol), and palladium (II) acetate (1 mg). The reaction mix was kept overnight at room temperature and followingly poored on aqueous phosphate buffer, pH 7: acetone (1:1 v/v 50 mL, 0.25 M $K_2HPO_4$), stirred for 5 min, and additioned with ethanedithiol (0.5 mL). After 20 min of stirring, $CHCl_3$ (30 mL) and acetic acid were added to acidify the aqueous phase, and the mixture was stirred for 1 h before the phases were separated. The aqueous layer was extracted (2 x 30 mL) with $CHCl_3$. The combined organic layers were dried over $Na_2SO_4$ and evaporated to dryness. The orange residue was purified by chromatography on Silica Gel (Packed in with $CHCl_3$-0.5% HOAc, sample loaded in $CHCl_3$) with elution from 0.5% HOAc-$CHCl_3$ to ethyl acetate-0.5% HOAc. The

combined fractions were evaporated and subjected to trituration with EtOH-$H_2O$. 22.5 mg (34% yield) of a whitish-pink solid was obtained. $^1$H-NMR ($CDCl_3$-$CD_3OD$ 1:1, ppm): 3.54 (m, partially obscured by solvent), 6.49 (d, 2H, *J* = 9 Hz), 6.62 (d, 2H, *J* = 9 Hz), 7.20 (d, *J* = 6 Hz, H-6), 7.65 (dd, *J* = 6 and 2 Hz, H-4,5 ), 8.00 (d, *J* = 6 Hz, H-3), 9.89 (s, 2H, OH). ESI-HRMS: $[M+H]^+$: 664.8541. Calcd for $C_{24}H_{19}O_5As_2S_4$ 663.8547.

**4',5'-Bis(1,2,3-dithioarsolan-2-yl)-2',7'-difluorofluorescein, F2-FlAsH-EDT$_2$.**

Crude 2',7'-difluoroFluorescein-4',5'-bis(mercuric trifluoroacetate) (0.2 g, 0.2 mmol) was suspended in dry NMP (3 mL) under Ar and treated with arsenic trichloride (0.34 mL, 4.0 mmol), DIPEA (0.28 mL, 1.6 mmol), and palladium (II) acetate (1 mg), following the procedure described above. Purification was carried out by column chromatography packed with Florisil, eluted with $CH_2Cl_2$-0.5% HAcO gradient up to AcOEt-0.5% HAcO. The combined fractions were evaporated and subjected to trituration with EtOH-$H_2O$. 13.4 mg (9.8% yield) of an orange solid was obtained. $^1$H-NMR ($CDCl_3$, ppm): 3.61 (m, 8H), 6.42 (d, 2H, *J* = 10 Hz ), 7.21 (d, 1H, *J* = 5 Hz, H-3 ), 7.65 - 7.74 (m, 2H, H-4, 5), 8.03 (d, 1H, *J* = 5 Hz, H-6), 10.13 (s, 2H, OH). ES-HRMS $[M+H]^+$: 700.8358 Calcd for $C_{24}H_{17}F_2O_5As_2S_4$ 700.8358.

**4',5'-Bis(1,2,3-dithioarsolan-2-yl)-3,4,5,6-tetrafluorofluorescein, F4-Flash-EDT$_2$.**

Crude 3,4,5,6-tetrafluoroFluorescein-4',5'-bis(mercuric trifluoroacetate) (0.3 g, 0.3 mmol) was suspended in dry NMP (3 mL) under Ar and treated with arsenic trichloride (0.5 mL, 3.0 mmol), DIPEA (0.4 mL, 2.4 mmol), and palladium (II) acetate (1 mg), following the procedure described above. Purification was carried out by chromatography on a Florisil column, eluted with $CH_2Cl_2$-0.5% HAcO gradient up to AcOEt-0.5% HAcO. The combined fractions were evaporated and subjected to trituration with EtOH-$H_2O$. $^1$H-NMR ($CDCl_3$-$CD_3OD$, 1:1, ppm): 3.49 (m, partially obscured by solvent), 7.06 (d, 2H, *J* = 8.6 Hz), 7.25 (d, 2H, *J* = 8.6 Hz), 10.50 (s, 2H, OH). ES-HRMS: $[M+H]^+$: 736.8168. Calcd for $C_{25}H_{15}As_2F_5O_5S_4$ 736.8170.

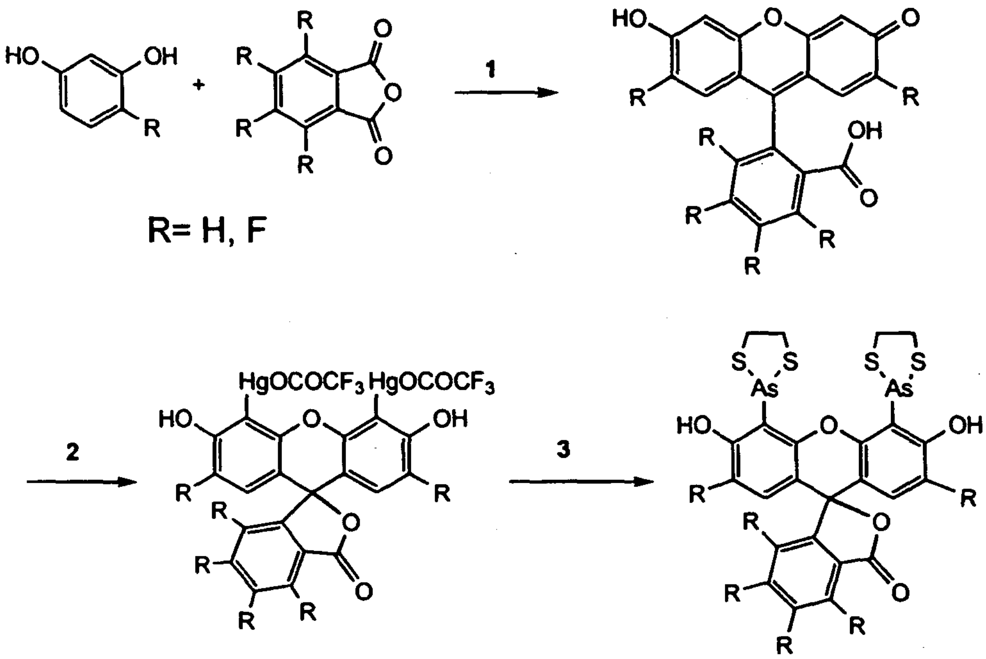


1- $CH_3SO_3H$, 80-85˚C, 48 hs
2- HgO, TFA, r.t., 12 hs
3- i) $AsCl_3$, DIPEA, $Pd(AcO)_2$, NMP; r.t., overnight; ii) $K_2HPO_4$ 1.4 M: Acetone (1:1), EDT

**3 - Spectroscopic Properties**

**3.1 - In vitro labeling of a model peptide with fluorinated FlAsH derivatives**

To a solution of the biarsenical compound in buffer HEPES 20 mM, 1 mM 2-ME, pH 7.4, at a concentration of 0.1 μM and with a slight excess of EDT, was added the model target peptide P12 (FLNCCPGCCMEP) to a final concentration of 1 μM from a stock solution in the same buffer and treated with 10 mM TCEP to reduce any disulfide bond. Emission spectra were taken after two hours of formation of the complex. Fig. 4 depicts the emision spectra of the F2FlAsH, F4FlAsH and FlAsH at equal concentration, with excitation at 490 nm. A 4-fold enhanced fluorescence emission of F2FlAsH compared to FlAsH can be observed.

**3.2 - Absorption spectra of biarsenical compounds and pH dependence in the physiological range**

The absorption spectra of 20 μM solutions of F2FlAsH, F4FlAsH and FlAsH-$EDT_2$, in 20 mM phosphate buffer, at different pH values, are shown in Fig. 5. In all cases, the absorbance and the corresponding emission, increases with the increase of pH.

**3.3- Fluorescence lifetimes**

The fluorescence lifetimes of the complexes obtained as described in section 3.1, were determined by TCSPC. The corresponding values for each parent fluorescein were also determined in 20 mM buffer HEPES, pH 7.4 for comparison.

| P12-Complex | t (ns) | Fluorophore | t (ns) |
|---|---|---|---|
| FlAsH | 4.88 | Fluorescein | 4.07 |
| F2FlAsH | 4.78 | 2',7'-difluorofluorescein | 4.02 |
| F4FlAsH | 5.18 | 3,4,5,6-tetrafluorofluorescein | 4.13 |

### 4 - Photostability

Samples containing 10 μM of the biarsenical compounds and 10 μM of the model peptide P12, were incubated at room temperature for 1 h. Each solution of the FlAsH-peptide complex was irradiated for 120 min as previously described (see General Procedures).

Values of $k_{b1}$ were calculated from the monoexponential fitting of the experimental photobleaching decay, according to the model described in (Eq. 1) for the intermediate irradieation regime.[13]

$$k_{fit} = k_{bl} \cdot \tau \cdot \psi \cdot \sigma \cdot t \qquad Eq.\ 1$$

where τ(s) is the lifetime, ψ is the irradiance (photons $cm^{-2}$ $s^{-1}$) and σ is the molecular absorption cross-section ($cm^2$ $molecule^{-1}$). Inasmuch as a broad excitation bandwidth was used, σ was computed as a mean value over the spectral distribution function of photon flux and corrected by the spectral integral with the corresponding absorption spectra, including the irradiation source and filter.

Photobleaching of F2FlAsH, F4FlAsH and FlAsH complexes with P12 lead to a residual fluorescence with maxima at 516, 538 and 519.5 nm, respectively.

### 5 - FRET determinations

A 0.8 μM solution in 20 mM buffer HEPES, 0.1 M NaCl, 1 mM 2-ME, pH 7.4 of the complex F2FlAsH-P12-biotin was incubated with an equimolar solution of streptavidin and titrated with increasing amounts of F4FlAsH-P12-biotin in a quartz cuvette equipped with a magnetic stirrer. Each addition was followed by measurement of the emission intensity using 490 nm as the excitation wavelength. FRET efficiency was determined according to.

### 6 - Steady state anisotropy

Steady state emission anisotropy of 1 μM solutions of F2FlAsH-P12-biotin and F4FlAsH-P12-biotin in 20 mM in buffer HEPES, NaCl 0.1M, 1 mM 2-ME, pH 7.4 was determined abased on eq. 2, were G is the correction factor for the bias in the detection of the two polarized components $I_{\Box}, I_{\perp}$

$$r = \frac{(I_{\Box}/I_{\perp}) - G}{I_{\Box}/I_{\perp} + 2G} \qquad Eq.2$$

## Claims

**1.** A fluorogenic dye having the formula

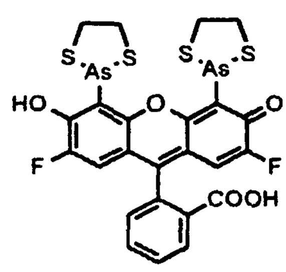

and salts of said fluorogenic dye.

**2.** A complex of the fluorogenic dye of claim 1 and a protein with a tetracystein motif.

**3.** A method of synthesizing the fluorogenic dye of claim 1 or claim 8, comprising the steps of

a) reacting

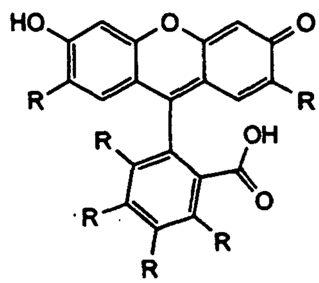

with HgO to form

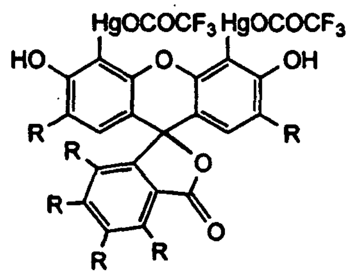

b) reacting

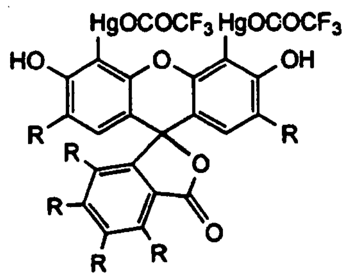

with $AsCl_3$ and EDT to form

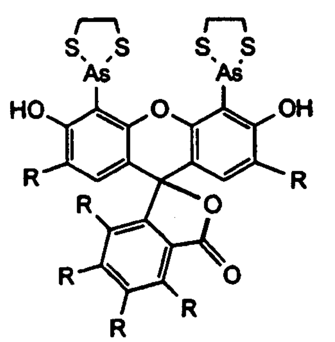

, wherein R = H, F.

4. A method for examining a sample comprising the steps

- contacting the fluorogenic dye of claim 1 with a sample
- measuring fluorescence.

5. The method of claim 4, wherein the sample comprises a protein.

6. The method of claim 4 or claim 5, wherein the sample is a living biological cell.

7. Use of the fluorogenic dye of claim 1 for fluorescence resonance energy transfer, FRET, experiments.

8. The use of claim 7, wherein the dye of claim 1 and a fluorogenic dye having the formula

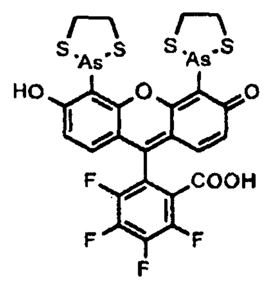

are involved and forming a FRET pair, one dye acting as a FRET donor and the other dye acting as a FRET acceptor.

**Patentansprüche**

1. Fluoreszenzfarbstoff mit der Formel

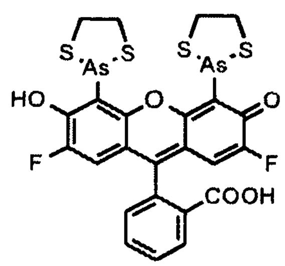

sowie Salze dieses Fluoreszenzfarbstoffs.

2. Komplex aus dem Fluoreszenzfarbstoff gemäß Anspruch 1 und einem Protein mit einem Tetracyctein-Motiv.

3. Verfahren zum Synthetisieren des Fluoreszenzfarbstoffs gemäß Anspruch 1 oder Anspruch 8, umfassend die Schritte:

a) reagieren Lassen von

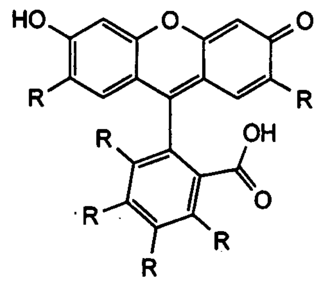

mit HgO zur Bildung von

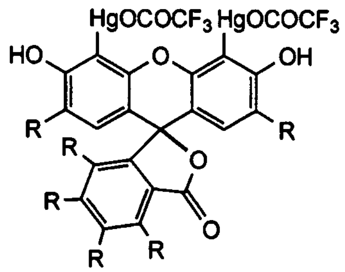

b) reagieren Lassen von

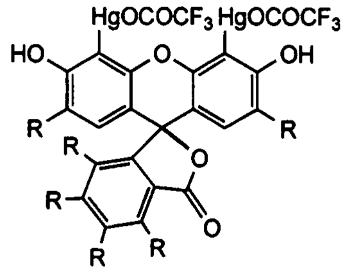

mit $AsCl_3$ und EDT zur Bildung von

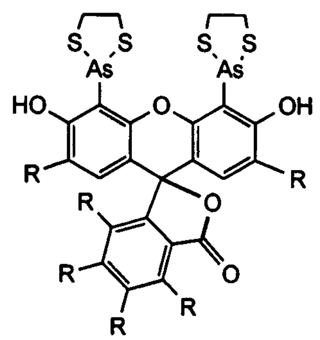

, wobei R = H, F.

4. Verfahren zum Untersuchen einer Probe, umfassend die Schritte:

- In-Kontakt-Bringen des Fluoreszenzfarbstoffs gemäß Anspruch 1 mit der Probe
- Messen von Fluoreszenz.

5. Verfahren nach Anspruch 4, wobei die Probe ein Protein umfasst.

6. Verfahren nach Anspruch 4 oder Anspruch 5, wobei die Probe eine lebende biologische Zelle ist.

7. Verwendung des Fluoreszenzfarbstoffs gemäß Anspruch 1 für Fluoreszenzresonanz-Energietransfer-, FRET-, Experimente.

8. Verwendung gemäß Anspruch 7, wobei der Farbstoff gemäß Anspruch 1 und ein Fluoreszenzfarbstoff mit der Formel

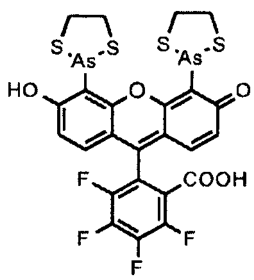

beteiligt sind und ein FRET-Paar bilden, bei dem ein Farbstoff als ein FRET-Donor und der andere Farbstoff als ein FRET-Akzeptor wirkt.

## Revendications

1. Un colorant fluorogénique de formule

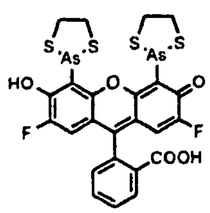

et les sels dudit colorant fluorogénique.

2. Un complexe du colorant fluorogénique de la revendication 1 et une protéine possédant un motif tétracystéine.

3. Une méthode de synthèse du colorant fluorogénique de la revendication 1 ou 8 comprenant les étapes suivantes :

a) la réaction de

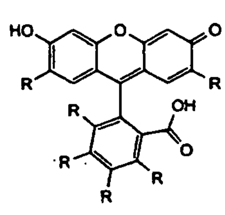

avec HgO pour former

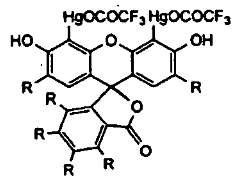

b) la réaction de

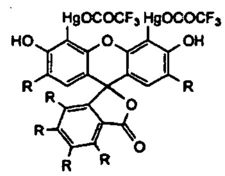

avec $ASCl_3$ et EDT pour former

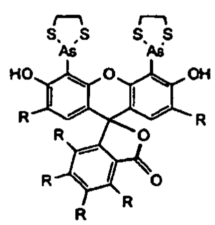

,

où R = H, F.

**4.** Une méthode d'examen d'échantillon comprenant

- la mise en contact du colorant fluorogénique de la revendication 1 avec un échantillon
- la mesure de la fluorescence.

**5.** La méthode de la revendication 4, avec une protéine dans l'échantillon.

**6.** La méthode de la revendication 4 ou de la revendication 5, avec une cellule biologique vivante comme échantillon.

**7.** L'utilisation du colorant fluorogénique de la revendication 1 pour des expériences de transfert d'énergie par résonance de fluorescence (FRET).

**8.** L'utilisation de la revendication 7, en associant le colorant de la revendication 1 et un colorant fluorogénique de formule

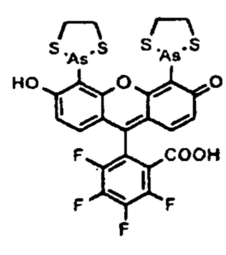

pour constituer une paire FRET, l'un des colorants agissant en tant que donneur FRET et l'autre en tant qu'accepteur FRET.

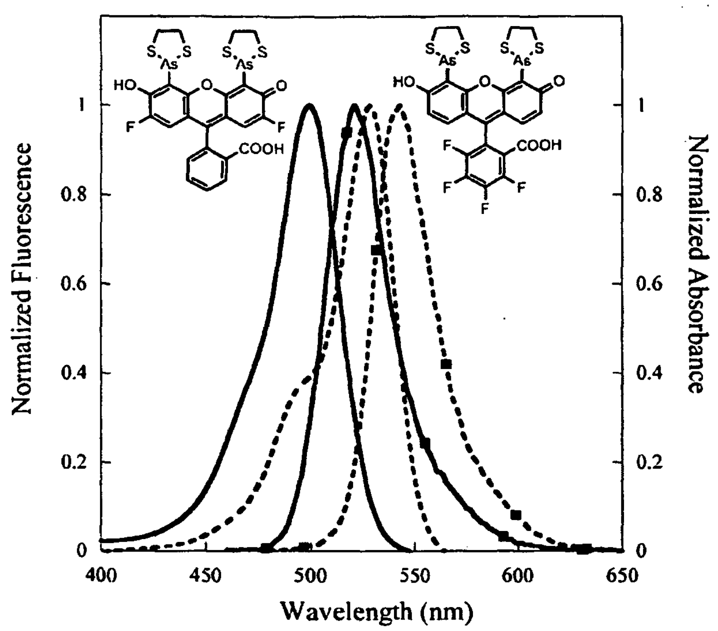


*Fig. 1*

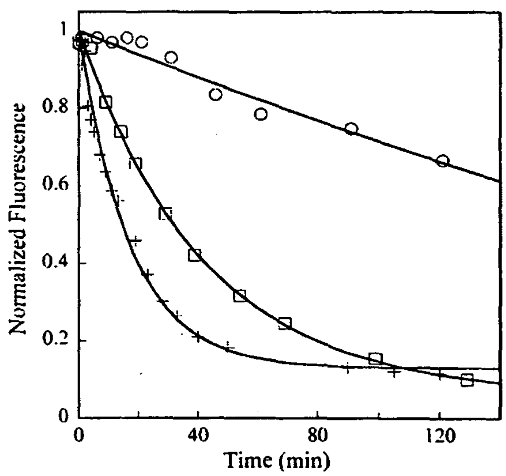


*Fig. 2*

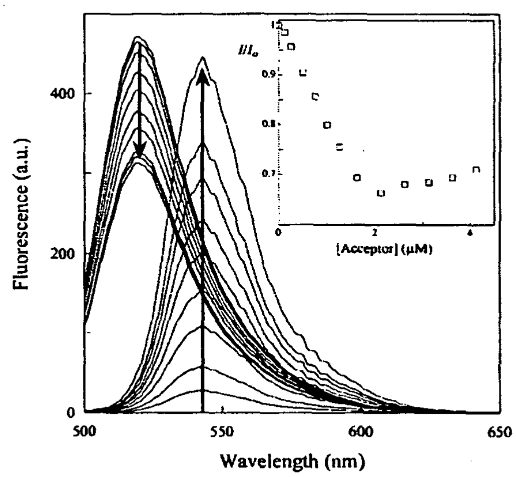


*Fig. 3*

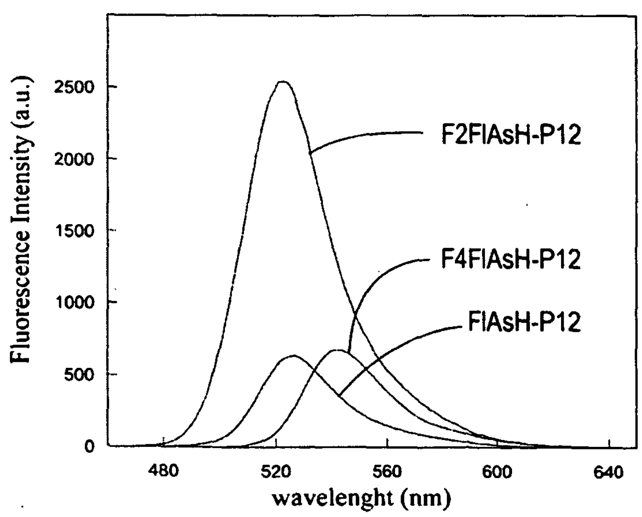


*Fig. 4*

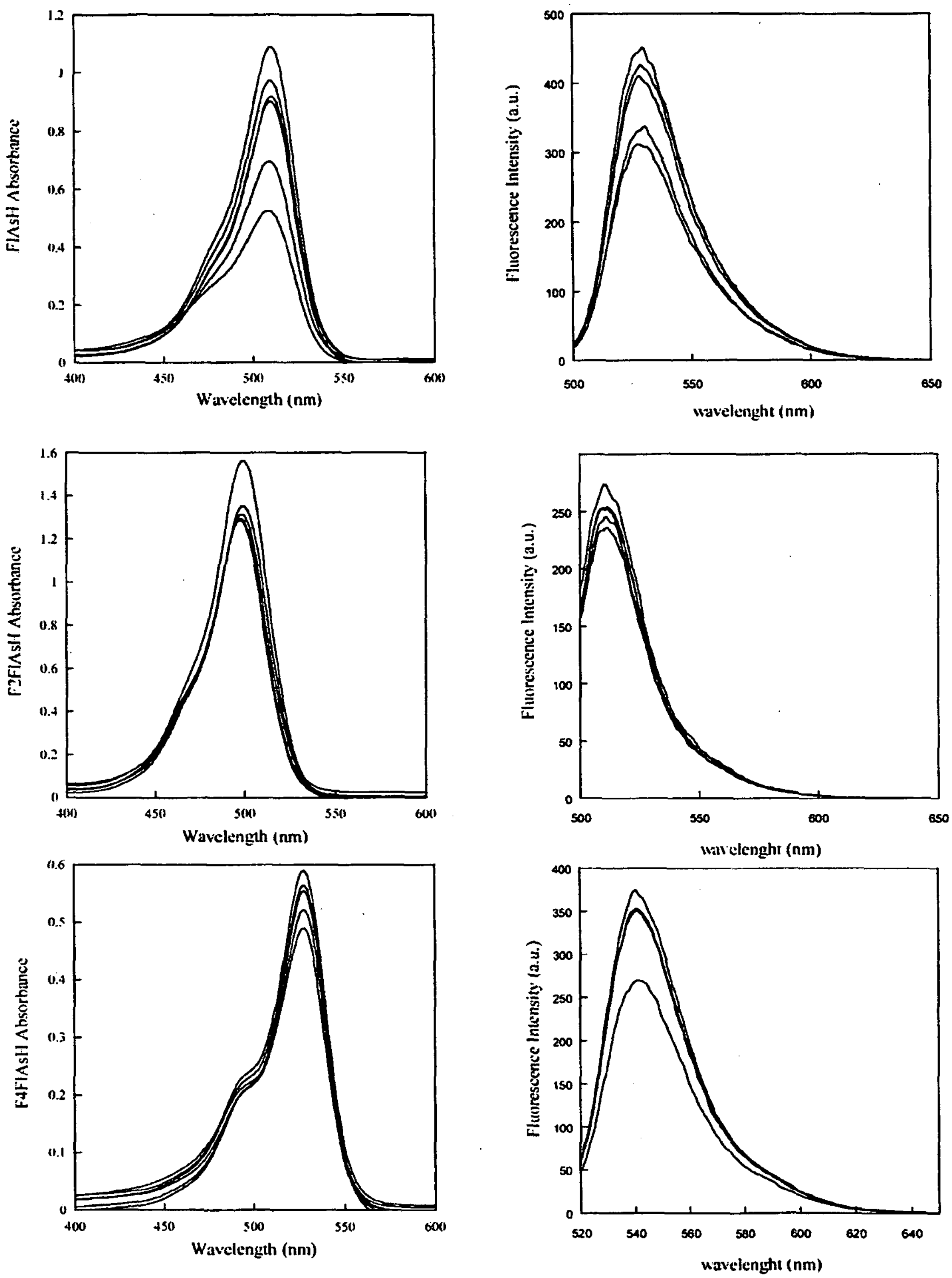
FlAsH Absorbance
Wavelength (nm)
Fluorescence Intensity (a.u.)
wavelenght (nm)
F2FlAsH Absorbance
Wavelength (nm)
Fluorescence Intensity (a.u.)
wavelenght (nm)
F4FlAsH Absorbance
Wavelength (nm)
Fluorescence Intensity (a.u.)
wavelenght (nm)

*Fig. 5*

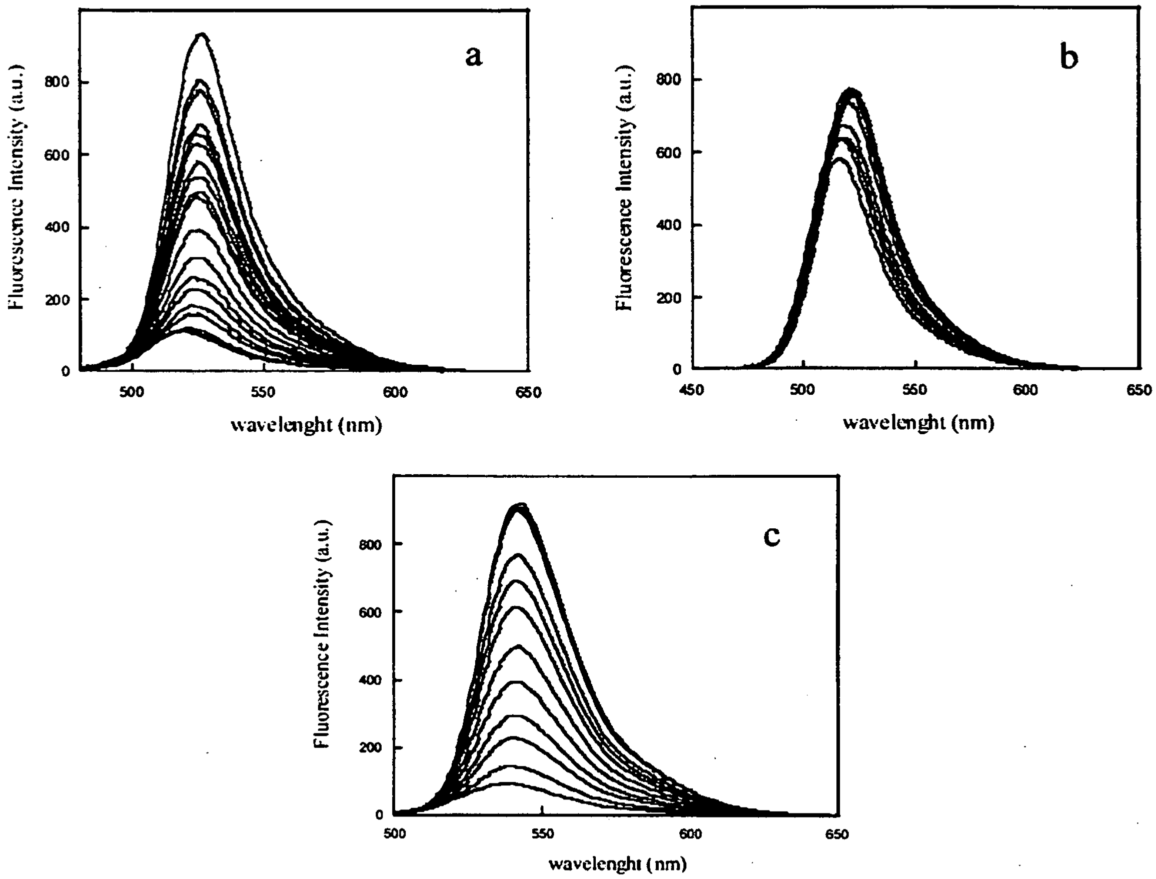
a
Fluorescence Intensity (a.u.)
0
200
400
600
800
500
550
600
650
wavelenght (nm)
b
Fluorescence Intensity (a.u.)
0
200
400
600
800
450
500
550
600
650
wavelenght (nm)
c
Fluorescence Intensity (a.u.)
0
200
400
600
800
500
550
600
650
wavelenght (nm)

*Fig. 6*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6008378 A

### Non-patent literature cited in the description


- **Griffin, B. A. ; Adams, S. R. ; Tsien, R. Y.** *Science,* 1998, vol. 281, 269-272
- **Sosinsky, G. ; Gaietta, G. ; Giepmans, B. ; Deerinck, T. ; Adams, S. ; Hand, G. ; Mackey, M. ; Terada, M. ; Smock, A. ; Tsien, R. Y.** *Biophys. J.,* 2005, vol. 88, 31A-32A
- **Tour, O. ; Meijer, R. M. ; Zacharias, D. A. ; Adams, S. R. ; Tsien, R. Y.** *Nat. Biotechnol.,* 2003, vol. 21, 1505-1508
- **Hoffmann, C. ; Gaietta, G. ; Bunemann, M. ; Adams, S. R. ; Oberdorff-Maass, S. ; Behr, B. ; Vilardaga, J. P. ; Tsien, R. Y. ; Eisman, M. H. ; Lohse, M. J.** *Nat. Methods,* 2005, vol. 2, 171-176
- **Jares-Erijman, E. A. ; Jovin, T. M.** *Nat. Biotechnol.,* 2003, vol. 21, 1387-1395
- **Selvin, P.R.** *Annu. Rev. Biophys. Biomol. Struct.,* 2002, vol. 31, 275-302
- **Martin, B. R. ; Giepmans, B. N. G. ; Adams, S. R. ; Tsien, R. Y.** *Nat. Biotechnol.,* 2005, vol. 23, 1308-1314
- **Adams, S. R. ; Campbell, R. E. ; Gross, L. A. ; Martin, B. R. ; Walkup, G. K. ; Yao, Y. ; Llopis, J. ; Tsien, R. Y.** *J. Am. Chem. Soc.,* 2002, vol. 124, 6063-6076
- **Sun, W. C. ; Gee, K. R. ; Klaubert, D. H. ; Haugland, R. P.** *J. Org. Chem.,* 1997, vol. 62, 6469-6475
- **Lindqvist, L.** *Arkiv. Kemi,* 1961, vol. 16, 79-138
- **Sun, W. C. ; Gee, K. R. ; Klaubert, D. H. ; Haugland, R. P.** Synthesis of fluorinated fluoresceins. *J. Org. Chem.,* 1997, vol. 62 (19), 6469-6475
- **Adams, S. R. ; Campbell, R. E. ; Gross, L. A. ; Martin, B. R. ; Walkup, G. K. ; Yao, Y. ; Llopis, J. ; Tsien, R. Y.** New biarsenical Ligands and tetracysteine motifs for protein labeling in vitro and in vivo: Synthesis and biological applications. *J. Am. Chem. Soc.,* 2002, vol. 124 (21), 6063-6076
- **Jovin, T. M. ; Arndt-Jovin, D. J. ; Marriott, G. ; Clegg, R. M. ; Robert-Nicaud, M. ; Schormann, T.** *Optical Microscopy for Biology,* 1990, 575-602